## Europäisches Patentamt
## European Patent Office
## Office européen des brevets

(11) Publication number: **0 168 172**
**B1**

# (12) EUROPEAN PATENT SPECIFICATION

(45) Date of publication of patent specification: **16.03.88**

(51) Int. Cl.⁴: **A 61 B 17/06,** A 61 L 17/00

(21) Application number: **85304073.1**

(22) Date of filing: **10.06.85**

(54) **A dispensing pack for surgical sutures.**

(30) Priority: **14.06.84 US 620413**

(43) Date of publication of application:
**15.01.86 Bulletin 86/03**

(45) Publication of the grant of the patent:
**16.03.88 Bulletin 88/11**

(84) Designated Contracting States:
**AT BE CH DE FR GB IT LI LU NL SE**

(56) References cited:
**DE-A-2 618 662**
**DE-A-2 717 537**
**DE-A-2 914 480**
**DE-A-3 016 606**
**DE-C- 637 275**
**FR-A-1 574 869**
**FR-A-2 455 880**
**GB-A- 970 685**
**US-A-2 692 676**
**US-A-4 034 763**
**US-A-4 034 850**
**US-A-4 261 463**

(73) Proprietor: **W.L. GORE & ASSOCIATES, INC.**
**555 Paper Mill Road P.O. Box 9329**
**Newark Delaware 19711 (US)**

(72) Inventor: **Lincoln, Jay Perry**
**R.R. No. 3, Box 24**
**Flagstaff Arizona 86001 (US)**
Inventor: **Owens, William Michael**
**1901 North Rain Tree**
**Flagstaff Arizona 86001 (US)**
Inventor: **Ferguson, Douglas Melvin**
**R.R. No. 1, Box 1020**
**Flagstaff Arizona 86001 (US)**

(74) Representative: **Taylor, Derek George et al**
**MATHISEN, MACARA & CO. The Coach House**
**6-8 Swakeleys Road**
**Ickenham Uxbridge UB10 8BZ (GB)**

Courier Press, Leamington Spa, England.

## Description

This invention relates to a dispensing pack for surgical sutures.

Packages for surgical sutures having needles attached to one or both ends are constructed according to the nature of the suture material and to how the sutures will be used.

Many of the prior packages are not suitable in that crimping, flattening, tangling, or knotting may occur during loading, handling, or removing a suture from the package.

It is known, for example from FR—A—2 455 880, to provide a suture pack within which a suture is arranged in a single loop, but this requires the use of a very long pack, albeit hinged at its centre.

It is also known, from DE—A—2 914 480, to provide a dispensing pack for surgical sutures comprising a first panel forming a closure affixed to a second panel, the two panels defining therebetween a space within which a suture is protectively confined and arranged in a plurality of loops, one said panel having a recess extending inwardly from an edge thereof, a needle attached to said suture and extending across said recess, and needle-supporting means disposed adjacent said recess for supporting said needle. In this pack, and also in a similar pack disclosed in US—A—4 034 850, the suture is wound around supporting posts and it is therefore necessary to separate the panels in order to remove a suture.

According to the present invention there is provided a dispensing pack for surgical sutures comprising a first panel forming a closure affixed to a second panel, the two panels defining therebetween a space within which a suture is protectively confined and arranged in a plurality of loops, said second panel having a recess extending inwardly from an edge thereof, a needle attached to said suture and extending across said recess, needle-supporting means disposed adjacent said recess for supporting said needle, and locking means integrally moulded in said panels for interlocking said panels upon closure, the dimensions of said panels being such that said needle-supporting means are disposed beyond said first panel upon closure, characterized in that the first panel is hingedly affixed to the second panel, and said second panel has integrally moulded interconnecting compartments therein within each of which the suture extends in an unsupported loop, each compartment communicating with the next adjacent compartment through a slot within which the suture extends from one compartment to the next whereby, upon withdrawal of the needle, or a pair of needles attached to opposite ends of the suture, from the pack, the suture is withdrawn one loop at a time from the interconnected compartments without separation of the panels and without danger of entanglement.

By means of the suture dispensing pack according to this invention several advantages over prior art packages for PTFE sutures can be secured. The advantage of non-overlapping of PTFE suture strands can be secured by locating the suture in a sinusoidal manner as it extends between the interconnected compartments, and it can be drawn out without opening the panels of the pack. This is combined with holding of the needles in a fixed position in a unique orientation which presents the needles in such a way that they may be gripped from either side of the package by a needle holder. Right-handed or left-handed removal of the needle and suture is equally facile. The needles can be rendered immediately visible in the top of the pack on only a partial peeling back of a protective envelope and can be immediately gripped in the recess from either side of the pack and the suture easily withdrawn from the pack without further opening of the protective envelope.

In a preferred embodiment of the invention, the pack is molded in plastics material and has a first panel connected by molded plastic hinges along a major edge to a second panel along a portion of a major edge. Both panels and the connecting hinges are molded simultaneously as one piece. The first panel is flat and has linear access slots extending across the width of the panel disposed at even predetermined distances apart down its length with holes spaced in between the slots so as to receive and hold the tops of studs at the ends of dividers which form suture holding compartments in the second panel.

The second panel is longer than the first panel, the needle-holding portion extending beyond the first panel, has chamfered upper corners to ease insertion of the loaded folded pack into a protective envelope, an optional suture retainer slot cut into the left end of the upper edge, a U-shaped recess disposed in the centre of the upper edge, two plastic needle clips or holders disposed one on each side of the U-shaped recess and, evenly disposed below the U-shaped recess and needle clips, a series of interconnected separate molded suture compartments arranged on an even predetermined spacing in registration with the slots of the first panel when the first panel is folded over the second panel. The studs are slightly tapered (optionally) and extend above the surface of the second panel so as to penetrate the holes provided in the first panel on closure of the pack to hold the first panel firmly to the face of the second panel by tight pressure fit in the holes.

On loading, the needles attached to the suture are fixed in place across the U-shaped notch of the second panel behind the clips or in a set of slots, the suture disposed along the space provided along the interconnection channel of the suture compartment, the first panel folded over to cover the suture compartment of the second panel and affixed in place by interlocking tongue and groove or other suitable means molded into the edges of the two panels, as well as or as an alternative to the studs of the second panel fitting the holes in register with them on the first panel, and the suture disposed in sinusoidal configuration in the suture compartments with utilization of the access slots of the first panel.

The needle point always faces to the right, away from the suture channel into the suture holding compartments.

The invention will now be particularly described by way of example with reference to the accompanying drawings in which:—

Figure 1 is a front plan view of an unfolded plastic pack according to the invention shown containing a suture with a needle attached at each end, and showing compartments of the pack separated by dividers;

Figure 1A is a cross-sectional view of the pack taken through one divider between compartments, the pack being shown closed and locked;

Figure 1B is a broken perspective view of an end portion of the pack in which suture-bearing needles are shown held by integral plastic clips;

Figure 1C is a plan view of the end portion of the end portion of the pack showing a single needle with suture held by the plastic clips and the free end of the suture held in a slot in the end of the pack;

Figure 1D is a plan view of an end portion of a modified pack;

Figure 2 is a plan view of an alternative embodiment of the pack showing a suture bearing one attached needle, the needle held in place in a pair of slots formed at an end of the pack;

Figure 2A is a perspective partial view of the end of the pack of Figure 2 showing a needle held in a pair of slots;

Figure 3 is a plan view of a thin plastics separator which can be used to separate sutures in a multiply-loaded pack;

Figure 3A is an exploded perspective view of a multiply-loaded pack with one suture disposed on a base panel of the pack, one separator to be placed on it, and another suture to be placed on the separator;

Figure 4 is a perspective view of the end portion of a pack showing a partially peeled back inner protective envelope with the suture pack displaying the needles in readily available position; and

Figure 5 is a perspective view showing a plastic suture pack being inserted into an inner protective envelope which, after sealing is inserted into an outer clear envelope which is also sealed.

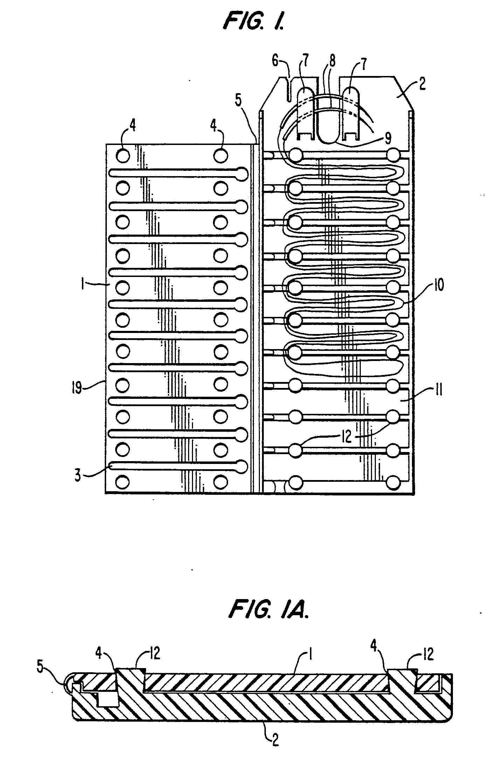

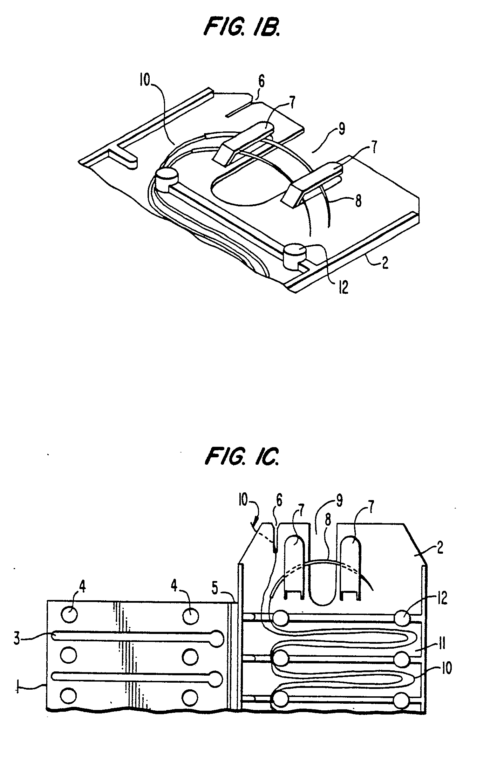

Referring now to the drawings, identical numerals are used for identical parts in each of the figures to aid in the description of the suture pack of the invention. Figure 1 shows in unfolded configuration (as it was molded) a two panel plastics pack according to the invention. The first panel 1 forms a cover which is joined by one or more integrally molded plastic hinges 5 to a second panel 2 which forms a base. When the cover panel is folded over on to base panel 2, it covers the suture holding compartment within the base panel 2 but leaves exposed the end portion of the base panel. This end portion contains clips 7 in which needles 8 are supported so that they extend across a U-shaped recess 9 in the end of panel 2.

The first panel 1 has a series of spaced slots 3 extending across its width at predetermined spacings with holes 4 disposed between the slots 3.

Slots 3 extend fully through panel 1. The holes 4 are in register with the molded studs 12 located adjacent the ends of dividers between the suture holding compartments 11 so that a tight pressure fit is obtained when the pack is closed and the two panels are held firmly together. Studs 12 are slightly tapered to assure firm holding in the holes 4.

The second panel 2 has an edge slot 6 which may optionally be utilized to anchor the end of a suture, the slot 6 extending parallel to the U-shaped recess 9. The clips 7 for supporting the needles 8 may be punched out from the body of panel 2 or optionally molded in place as panel 2 is being molded. A suture 10 extending between needles 8 is shown disposed in a sinusoidal configuration the suture lying in a long loop in each compartment and extending from each compartment 11 to the next via a slot at one end of each of the compartment dividers. Accordingly, upon removal of a needle from its supporting means, the suture can be drawn out of the pack without opening the pack.

Adjacent the ends of the dividers between suture holding compartments 11, the studs 12 are molded to extend above the surface of the remainder of panel 2 and extend slightly above the outer surface of panel 1 when in closed position covering a portion of the face of panel 2.

To close the pack, panel 1 is folded over on to panel 2 and locked by engagement of the studs 12 in the holes 4 as seen in Figure 1A. Panel 1 is shown optionally hinged 5 to panel 2.

Figure 1B shows more clearly how a pair of needles 8 attached to a suture 10 are held by the pair of integral plastic clips 7 across the recess 9 so that the needles can be conveniently reached for easy removal from either side of recess 9 of the pack, for example by a pair of forceps inserted into the recess to engage and grip a needle.

As an alternative to twin needles, the suture can extend from a single needle and the free end of the suture received in the slot 6 shown in Figures 1B, 1C and 1D.

Figure 1D depicts an alternative location for the thru-slots 3, namely at the bottom of the suture holding compartments 11 of panel 2 instead of in panel 1.

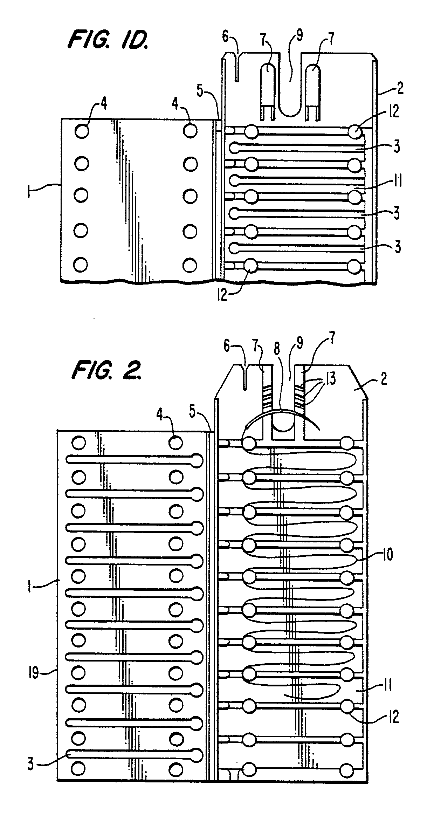

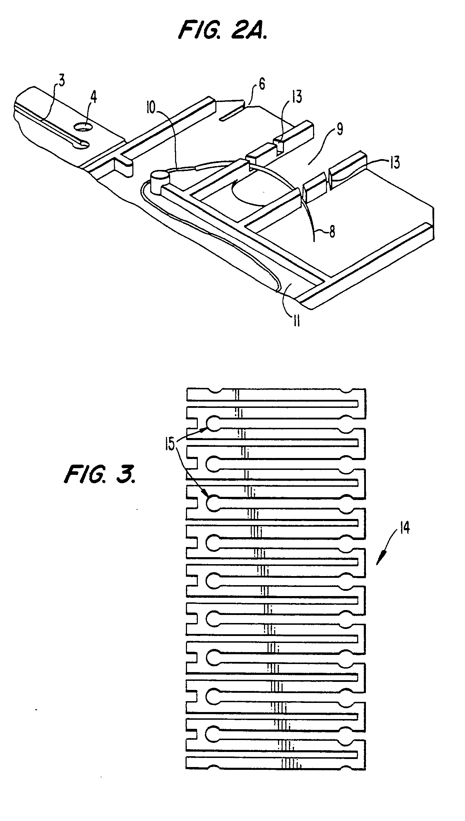

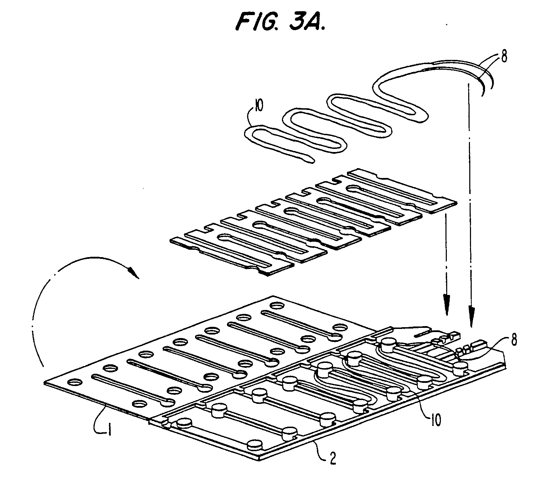

In the alternative embodiment of Figures 2 and 2A, ribs are molded along opposite sides of the recess 9 to contain pairs of shaped slots 13 which extend in predetermined curves across U-shaped recess 9, the curves chosen to match the curvature of the needles 8 to be placed in the pack. Where multiple sutures with attached needles are to be packaged, it is necessary in the case of PTFE sutures, for which the packs of the invention are designed, to be spaced apart from each other within the suture holding compartment 11 by thin plastic separators 14 as shown in Figure 3. The separators 14 contain slots extending alternately from opposite sides of the separator to give a serpentine or zig-zag configuration, one set of alternate slots containing apertures or enlargements 15 for engagement over the studs 12 of the base panel 2. The separators 14 are held in place in compartment 11 by careful sizing and registration of the enlargements 15 with the studs 12.

In the exploded perspective view of Figure 3A, the panels 1 and 2 are generally as shown in Figure 2 and one suture 10 is shown loaded in the pack, a thin plastics separator located above it, and the next suture to be loaded into the pack. The needles attached to this next suture would be disposed in an open shaped slot beyond that depicted as holding the needles of the first suture in place.

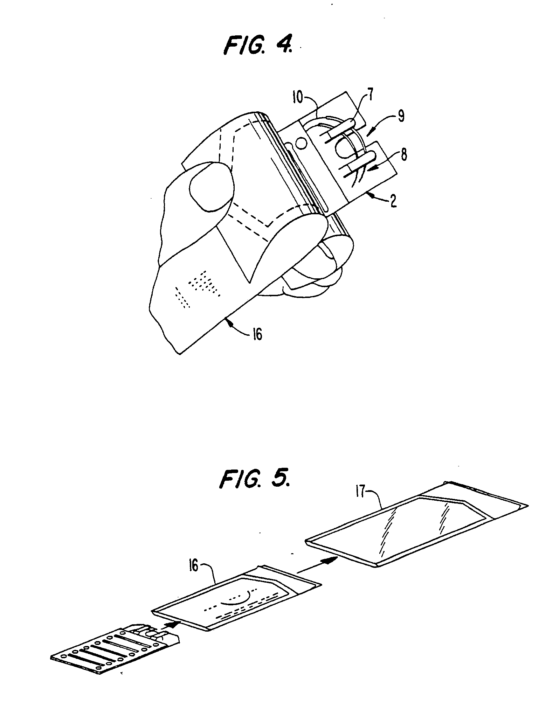

Figure 4 shows a pack according to the invention in which the needles 8 are exposed for ready use by partially peeling back a protective envelope 16. The needles 8 are completely visible either on the side of the pack on which they are held in place or through the transparent plastic panel 2 of the pack.

Figure 5 shows a suture and needle-loaded pack according to the invention which is ready to be placed inside inner protective envelope 16. Envelope 16 is sealed, then inserted inside clear outer protective envelope 17, envelope 17 is sealed, the packaged packs are cartoned, and then sterilized with, for instance, ethylene oxide gas. Printed indicia regarding contents and instructions for use can be disposed on the inner protective envelope 16.

The plastics suture pack can be manufactured from injection moldable plastics known in the art, such as polypropylene, polyethylene, polysulfone and other polymers. The suture separators utilized with multiple suture loadings may be cut from plastic sheets or molded from common plastics such as those cited above. The inner and outer protective envelopes are usually of heat-sealable thermoplastic polymers, such as, for instance, polyethylene, polypropylene, polyvinyl acetate-ethylene copolymer, or Tyvek (Du Pont de Menours trademark) diolefin polymer, which may also be in the form of composites with paper, aluminum foil, or other appropriate materials. It is preferred that the inner envelope be a composite bearing any required printed indicia and that the outer envelope be of a clear heat-sealable thermoplastic material. The porous expanded polytetrafluoroethylene utilized as suture material is that referred to above, and manufactured by W. L. Gore & Associates, Inc.

## Claims

1. A dispensing pack for surgical sutures comprising a first panel (1) forming a closure affixed to a second panel (2), the two panels defining therebetween a space within which a suture (10) is protectively confined, and arranged in a plurality of loops, said second panel having a recess (9) extending inwardly from an edge thereof, a needle (8) attached to said suture and extending across said recess, needle-supporting means disposed adjacent said recess for supporting said needle, and locking means (12, 4) integrally moulded in said panels (1, 2) for interlocking said panels upon closure, the dimensions of said panels being such that said needle-supporting means are disposed beyond said first panel upon closure, characterised in that the first panel (1) is hingedly affixed to the second panel (2), and said second panel (2) has integrally moulded interconnecting compartments (11) therein within each of which the suture extends in an unsupported loop, each compartment communicating with the next adjacent compartment through a slot within which the suture extends from one compartment to the next whereby, upon withdrawal of the needle, or a pair of needles attached to opposite ends of the suture, from the pack, the suture is withdrawn one loop at a time from the interconnected compartments without separation of the panels and without danger of entanglement.

2. A pack according to claim 1 characterised in that a separator shaped to correspond approximately to that of said suture holding compartments is disposed between two sutures disposed in said space.

3. A pack according to claim 1 characterised in that said recess is not overlapped by the first panel and the needle is exposed to be gripped by forceps or other means approaching the needle from opposite sides of said second panel.

4. A pack according to any preceding claim characterised in that the suture (11) is comprised of porous expanded polytetrafluoroethylene.

5. A pack according to any preceding claim characterised in that the compartments (11) are separated by dividers, each divider having a said slot adjacent one end thereof through which the compartments are interconnected and through which the suture (10) can extend between compartments in a sinusoidal manner.

6. A pack according to any preceding claim characterised in that elongate slots are molded into the first panel (1) of said pack to correspond with each of said compartments (11).

7. A pack according to any preceding claim characterised in that the needle-supporting means comprises integrally formed plastics clips (7) on either side of said recess (9).

8. A pack according to any one of claims 1 to 6 characterised in that the needle-supporting means comprise, for each needle, two slots (13) disposed on opposite sides of said recess (9) and curved to match the curvature of the needle.

9. A pack according to any preceding claim characterised in that the panels are secured together by interlocking means which comprises shaped apertures (4) disposed in a predetermined pattern on one panel which fit over and interlock with integrally molded studs (12) of a corresponding shape to said apertures disposed on the other panel to register with said apertures when said panels of the pack have been brought together.

10. A pack according to any preceding claim characterised in that it has been sealed into one or more plastic protective envelopes (16, 17) and the whole sterilized.

11. A pack according to any preceding claim characterised in that it has been molded from polypropylene, polyethylene, polysulfone, polystyrene or copolymers of styrene, polyvinyl chloride, acrylonitrile-butadiene-styrene polymers, acrylics, cellulosics, polyamide polymers, acetal, polycarbonate, fluoroolefin polymers, or silicone rubber.

## Patentansprüche

1. Ausgabepackung für chirurgisches Nähmaterial, mit einer ersten Platte (1) die einen an einer zweiten Platte befestigten Verschluß bildet, wobei diese beiden Platten einen dazwischenliegenden Raum umgrenzen, in dem ein Nähmaterial (10) geschützt eingeschlossen ist und in einer Mehrzahl von Schliefen angeordnet ist, und wobei die zweite Platte mit einer Ausnehmung (9) versehen ist, welche sich von einem Rand dieser Platte nach innen erstreckt, welche Packung weiter eine an Nähmaterial befestigte Nadel (8), die sich quer über die genannte Ausnehmung erstreckt, aufweist, und mit Nadelhaltemitteln zum Halten dieser Nadel versehen ist, welche dieser Ausnehmung benachbart angeordnet sind, und weiter in die genannten Platten (1, 2) integal eingeformte Verriegelungsmittel (12, 4) für ein miteinander Verriegeln dieser Platten nach dem Schließen aufweist, wobei die Abmessungen dieser Platten so sind, daß die Nadelhaltemittel nach dem Schließen außerhalb der genannten ersten Platte angeordnet sind, dadurch gekennzeichnet, daß die erste Platte (1) mit der zweiten Platte (2) scharnierartig verbunden ist und die zweite Platte (2) integral eingeformte, untereinander verbundene Abteile (11) aufweist, wobei in jedem dieser Abteile das Nähmaterial in einer ununterstützen Schleife verläuft und jedes Abteil mit dem nächsten benachbarten Abteil durch einen Schlitz in Verbindung steht, in dem das Nähmaterial von einem Abteil zum nächsten verläuft, wobei auf ein Wegnehmen der Nadel oder eines Paares von Nadeln, welche an entgegengesetzten Enden des Nähmaterials befestigt sind, von der Packung das Nähmaterial in einzelnen Schleifen aus den miteinander in Verbindung stehenden Abteilen herausgezogen wird, ohne die Platten voneinander zu trennen und ohne Gefahr eines Verwickelns.

2. Packung nach Anspruch 1, dadurch gekennzeichnet, daß ein Separator vorgesehen ist, der ungefähr der Form der das Nähmaterial haltenden Abteile entsprechend geformt ist und zwischen zwei Nähfäden, welche in diesem Raum untergebracht sind, angeordnet ist.

3. Packung nach Anspruch 1, dadurch gekennzeichnet, daß die Ausnehmung durch die erste Platte nicht überlappt ist, und daß die Nadel freiliegend angeordnet ist, um mit Pinzetten oder anderen Mitteln, die an die Nadel von gegenüberliegenden Seiten der zweiten Platte herangeführt werden, ergriffen werden kann.

4. Packung nach einem der vorhergehenden Ansprüche, dadurch gekennzeichnet, daß das Nähmaterial aus porösem, expandiertem Polytetrafluoräthylen zusammengesetzt ist.

5. Packung nach einem der vorhergehenden Ansprüche, dadurch gekennzeichnet, daß die Abteile (11) durch Teiler getrennt sind und jeder Teiler seinem einen Ende benachbart einen der genannten Schlitzt aufweist, durch die die Abteile miteinander verbunden sind und durch die das Nähmaterial (10) sinusartig zwischen den Abteilen verläuft.

6. Packung nach einem der vorhergehenden Ansprüche, dadurch gekennzeichnet, daß in die erste Platte (1) der Packung längliche Schlitze eingeformt sind, welche mit jedem der genannten Abteile (11) korrespondieren.

7. Packung nach einem der vorhergehenden Ansprüche, dadurch gekennzeichnet, daß die Nadeltragmittel seitlich der genannten Ausnenmung (9) integral angeformte Kunststoffklips (7) aufweisen.

8. Packung nach einem der Ansprüche 1 bis 6, dadurch gekennzeichnet, daß die Nadeltragmittel für jede Nadel zwei Schlitzt (13) aufweisen, welche an gegenüberliegenden Seiten der genannten Ausnehmung (9) angeordnet sind und passend zur Krümmung der Nadel gekrümmt sind.

9. Packung nach einem der vorhergehenden Ansprüche, dadurch gekennzeichnet, daß die Platten durch Verriegelungsmittel zusammengehalten sind, welche geformte öffnungen (4) aufweisen, die in einem vorbestimmten Muster auf einer Platte angeordnet sind und welche über integral angeformte Zapfen (12) passen und mit diesen Zapfen ineinandergreifen, welche Zapfen eine zu den genannten öffnungen entsprechende Form haben und auf der anderen Platte so angeordnet sind, daß sie mit den genannten öffnungen fluchten, wenn die genannten Platten der Packung zusammengefügt worden sind.

10. Packung nach einem der vorhergehenden Ansprüche, dadurch gekennzeichnet, daß sie in eine oder mehrere Kunststoffschutzhüllen (16, 17) eingesiegelt sind und das ganze sterilisiert ist.

11. Packung nach einem der vorhergehenden Ansprüche, dadurch gekennzeichnet, daß sie aus Polypropylen, Polyäthylen, Polysulfon, Polystyrol oder Copolymeren von Styrol, Polyvinylchlorid, Acrylnitril-Butatien-Styrolpolymeren, Acrylaten, Zellulosekunststoffen, Polyamidpolymeren, Azetalkunststoffen, Polykarbonat, Fluorolefinpolymeren, oder Silikongummi geformt ist.

## Revendications

1. Emballage de distribution pour sutures chirurgicales comprenant une première plaque (1) formant une fermeture fixée à une seconde plaque (2), les deux plaques définissant entre elles un espace à l'intérieur duquel une suture (10) est confinée en toute protection, et est agencée en plusieurs boucles, la seconde plaque comportant un évidement (9) s'étendant vers l'intérieur à partir d'un de ses bords, une aiguille (8) attachée à la suture et s'étendant à travers l'évidement, des moyens de support d'aiguille agencés à proximité de l'évidement pour supporter l'aiguille précitée et des moyens de blocage (12, 4) moulés d'une seule pièce dans les plaques (1, 2) pour verrouiller les plaques lors de la fermeture, les dimensions de ces plaques étant telles que les moyens de support d'aiguille sont agencés au-delà de la première plaque lors de la fermeture, caractérisé en ce que la première plaque (1) est fixée par articulation à la seconde plaque (2) et en ce que la seconde plaque (2) comporte des compartiments d'interconnexion moulés d'une seule pièce (11) à l'intérieur de chacun desquels la suture s'étend en une boucle non supportée, chaque compartiment communiquant avec le compartiment adjacent suivant par l'intermédiaire d'une fente à l'intérieur de laquelle la suture s'étend d'un compartiment au compartiment suivant de sorte que, lors de l'enlèvement de l'aiguille ou d'une paire d'aiguilles fixées à des extrémités opposées de la suture, de l'emballage, la suture est extraite sous la forme d'une boucle à un moment donné des compartiments interconnectés sans séparation des plaques et sans danger d'enchevêtrement.

2. Emballage suivant la revendication 1, caractérisé en ce qu'un élément séparateur façonné pour correspondre approximativement à celui des compartiments de support de suture est agencé entre deux sutures agencées dans l'espace précité.

3. Emballage suivant la revendication 1, caractérisé en ce que l'évidement n'est pas recouvert par la première plaque et l'aiguille est exposée pour être saisie par des pinces ou d'autres moyens approchant l'aiguille de côtés opposés de la seconde plaque.

4. Emballage suivant l'une quelconque des revendications précédentes, caractérisé en ce que la suture (11) est formée de polytétrafluoroéthylène expansé poreux.

5. Emballage suivant l'une quelconque des revendications précédentes, caractérisé en ce que les compartiments (11) sont séparés par des éléments diviseurs, chaque élément diviseur comportant une fente à proximité d'une de ses extrémités par laquelle les compartiments sont interconnectés et par laquelle la suture (10) peut s'étendre entre des compartiments d'une manière sinusoïdale.

6. Emballage suivant l'une quelconque des revendications précédentes, caractérisé en ce que des fentes allongées sont moulées dans la première plaque (1) de l'emballage pour correspondre à chacun des compartiments (11) précités.

7. Emballage suivant l'une quelconque des revendications précédentes, caractérisé en ce que les moyens de support d'aiguille comprennent des clips de matière plastique formés d'une seule pièce (7) de chaque côté de l'évidement (9).

8. Emballage suivant l'une quelconque des revendications 1 à 6, caractérisé en ce que les moyens de support d'aiguille comprennent, pour chaque aiguille, deux fentes (13) agencées sur des côtés opposés de l'évidement (9) et arrondies pour correspondre à la courbure de l'aiguille.

9. Emballage suivant l'une quelconque des revendications précédentes, caractérisé en ce que les plaques sont fixées ensemble par des moyens de verrouillage qui comprennent des ouvertures façonnées (4) agencées suivant un parcours préalablement déterminé sur une plaque, qui s'adaptent sur et se verrouillent avec des chevilles moulées d'une seule pièce (12) d'une forme correspondante à ces ouvertures, agencées sur l'autre plaque pour venir en regard de ces ouvertures lorsque les plaques de l'emballage ont été rapprochées.

10. Emballage suivant l'une quelconque des revendications précédentes, caractérisé en ce qu'il a été scellé dans une ou plusieurs enveloppes protectrices de matière plastique (16, 17) et le tout stérilisé.

11. Emballage suivant l'une quelconque des revendications précédentes, caractérisé en ce qu'il a été moulé dans du polypropylène, du polyéthylène, une polysulfone, du polystyrène ou des copolymères de styrène, du chlorure de polyvinyle, des polymères d'acrylontrile-butadiène-styrène, des matières acryliques, des matières cellulosiques, des polymères de polyamide, de l'acétal, du polycarbonate, des polymères fluorooléfiniques ou du caoutchouc de silicone.

## FIG. I.

## FIG. IA.

## FIG. IB.

## FIG. IC.

FIG. ID.

FIG. 2.

## FIG. 2A.

## FIG. 3.

0 168 172

# FIG. 3A.

## FIG. 4.

## FIG. 5.